# EUROPEAN PATENT SPECIFICATION

(11) **EP 3 228 253 B1**
(45) Date of publication and mention of the grant of the patent: **22.12.2021**
(21) Application number: 15864580.4
(22) Date of filing: 09.10.2015
(51) Int. Cl.: A61B 8/14, A61B 8/12

(54) **MEDICAL OBSERVATION DEVICE, METHOD FOR OPERATING MEDICAL OBSERVATION DEVICE, AND PROGRAM FOR OPERATING MEDICAL OBSERVATION DEVICE**
MEDIZINISCHE BEOBACHTUNGSVORRICHTUNG, VERFAHREN ZUM BETRIEB DER MEDIZINISCHEN BEOBACHTUNGSVORRICHTUNG UND PROGRAMM ZUM BETRIEB DER MEDIZINISCHEN BEOBACHTUNGSVORRICHTUNG
DISPOSITIF D'OBSERVATION MÉDICALE, PROCÉDÉ DE FONCTIONNEMENT DE DISPOSITIF D'OBSERVATION MÉDICALE, ET PROGRAMME DE FONCTIONNEMENT DE DISPOSITIF D'OBSERVATION MÉDICALE

(30) Priority: 02.12.2014 JP 2014244108
(43) Date of publication of application: 11.10.2017
(73) Proprietor: Olympus Corporation, Tokyo 192-8507 (JP)
(72) Inventor: HIBI, Yasushi, Tokyo 192-8507 (JP)
(74) Representative: Gunzelmann, Rainer
(86) International application number: PCT/JP2015/078839
(87) International publication number: WO 2016/088452

(56) References cited:
- JP-A- 2001 029 360
- JP-A- 2003 000 614
- JP-A- 2006 288 611
- JP-A- 2013 233 275
- US-A1- 2002 128 846
- US-A1- 2008 084 428
- US-A1- 2013 225 999
- US-A1- 2014 121 524

## Description

### Field

The present invention relates to a medical observation apparatus for observing an observation target using an ultrasound wave, for example, a method for operating the medical observation apparatus, and a program for operating the medical observation apparatus. Background

An ultrasound wave is used to observe the characteristics of living tissue or material as an observation target. Specifically, the ultrasound wave is transmitted to the observation target, and ultrasound echo reflected from the observation target is subjected to predetermined signal processing to obtain information on the characteristics of the observation target.

For diagnosis of the internal living tissue or the like with application of the ultrasound wave, an ultrasound endoscope is used which has an insertion section provided with an ultrasound transducer at a distal end. An operator such as a physician operates an operating unit in his/her hand after insertion of the insertion section in a body to obtain ultrasound echo using the ultrasound transducer, and performs diagnosis using information obtained based on the ultrasound echo (ultrasound image).

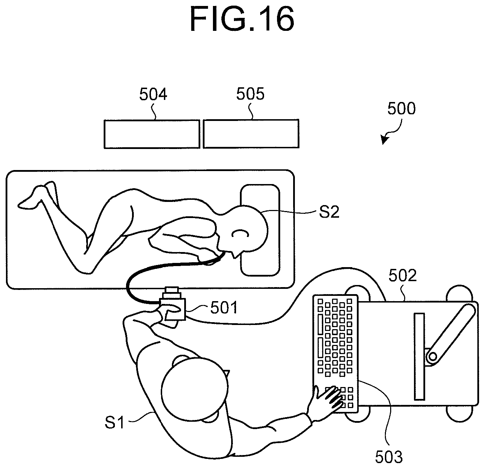

FIG. 16 is a schematic diagram illustrating a configuration of a conventional ultrasound diagnosis system. An ultrasound diagnostic system 500 illustrated in FIG. 16 includes an ultrasound endoscope 501 having an insertion section provided with an ultrasound transducer and an image sensor at a distal end, an image processing device 502 for generating an image obtained based on ultrasound echo and imaging signals obtained by the ultrasound endoscope 501, a keyboard 503 connected with the image processing device 502 to input signals such as instruction signals or the like, an ultrasound image monitor 504 for displaying the image based on the ultrasound echo, and an endoscopic image monitor 505 for displaying the image based on the imaging signals. An operator S1 inserts the insertion section of the ultrasound endoscope 501 into a subject S2, inputs the instruction signal through an operating unit provided at the keyboard 503 or the ultrasound endoscope 501, and adjusts an ultrasound image, specifically, rotation or movement of the image to make a diagnosis.

The operator S1 and the image processing device 502 (keyboard 503) are sometimes separated from each other depending on the layout of an examination room or the like. The separation of the operator S1 and the image processing device 502 (keyboard 503) unfortunately causes deterioration in operability. In order to address such a situation, a technique is disclosed in which voice recognition and foot control are combined to allow operation by the operator separated from the device (see Patent Literature 1, for example).

US 2001/128846 A1 concerns a system and method for controlling a medical device such as a medical imaging device. A verbal command for assigning a function of a medical imaging device is received from an operator. An additional verbal command from the operator assigns an input device to control the function of the selected medical imaging device. A system control and voice recognition processor then assigns the function specified by the verbal command to the input device selected by the additional verbal command. After the function of the medical device is assigned to the input device, the operator may control the selected function of the medical device with the selected input device.

### Citation List

### Patent Literature

Patent Literature 1: JP 2003-614 A

### Summary

### Technical Problem

The ultrasound echo has a property of displaying a cross-section of an observation position in a body, but when the observation position in the body is displaced, a cross-sectional observation image is differed, so that the observation position needs to be significantly maintained. However, in the above-mentioned technique of Patent literature 1, for example, when the operator looks aside from the ultrasound image monitor 504 to confirm the position of a foot control, during operation of the foot control for image adjustment, the operator may continue observation without recognizing displacement of the observation position.

The present invention has been made in view of the foregoing, and an object of the present invention is to provide a medical observation apparatus allowing an adjustment operation of an image on a monitor while watching the monitor, a method for operating the medical observation apparatus, and a program for operating the medical observation apparatus.

### Solution to Problem

In order to solve the above described problem and achieve the object, a medical observation apparatus according to the invention is configured to acquire a signal for generating an image of an observation target and to display an observation image based on the acquired signal. The medical observation apparatus includes the features of claim 1.

In the medical observation apparatus according to the above-described invention, the control unit is configured to control switching between the processing modes according to input of the first operation instruction signal, switching between the guide images according to the processing modes, or switching between the signal input functions according to the processing modes.

In the medical observation apparatus according to the above-described invention, the contactless signal input unit is configured to detect voice, gesture, eye gaze, or a brain wave, and the contact signal input unit includes at least one of a keyboard, a trackball, a mouse, a touch panel, a lever, a dial, a joystick, and a foot switch.

In the medical observation apparatus according to the above-described invention, the signal input functions according to the processing modes include continuous or discontinuous, or multistep or stepless contact operation.

In the medical observation apparatus according to the above-described invention, the processing modes include at least one of a rotation mode for rotating an image, a display area setting mode for setting a display area for image display within an observation area, a text input mode, a focus position setting mode, an enlargement or reduction mode, a region-of-interest setting mode, and a measurement mode.

In the medical observation apparatus according to the above-described invention, each of the guide images is an image representing a relationship between a direction of an operation by the contact signal input unit, and a direction of movement according to the at least one of the signal input functions in the at least one of the processing modes.

In the medical observation apparatus according to the above-described invention, the signal for generating the image of the observation target is a signal generated based on an echo signal that is obtained by converting an ultrasound echo into an electrical signal, the ultrasound echo being obtained by transmitting an ultrasound wave to the observation target and by reflecting the ultrasound wave from the observation target.

A method for operating a medical observation apparatus according to the invention is provided. The medical observation apparatus is configured to acquire a signal for generating an image of an observation target and to display an observation image based on the acquired signal. The method includes the features of claim 7.

A program for operating a medical observation apparatus according to the invention is provided. The medical observation apparatus is configured to acquire a signal for generating an image of an observation target and to display an observation image based on the acquired signal. The program causes the medical observation apparatus to execute the steps of claim 8.

### Advantageous Effects of Invention

According to the present invention, it is possible to effectively perform an adjustment operation of an image on a monitor while watching the monitor. Brief Description of Drawings
FIG. 1 is a block diagram illustrating a configuration of an ultrasound endoscopic system according to a first embodiment of the present invention.
FIG. 2 is a flowchart illustrating image adjustment operation performed by a processing device according to the first embodiment of the present invention.
FIG. 3 is a diagram illustrating the image adjustment operation performed by the processing device according to the first embodiment of the present invention.
FIG. 4 is a diagram illustrating the image adjustment operation performed by the processing device according to the first embodiment of the present invention.
FIG. 5 is a diagram illustrating image adjustment operation performed by a processing device according to a modification of the first embodiment of the present invention.
FIG. 6 is a diagram illustrating the image adjustment operation performed by a processing device according to a modification of the first embodiment of the present invention.
FIG. 7 is a flowchart illustrating image adjustment operation performed by a processing device according to a second embodiment of the present invention.
FIG. 8 is a diagram illustrating the image adjustment operation performed by the processing device according to the second embodiment of the present invention.
FIG. 9 is a diagram illustrating the image adjustment operation performed by the processing device according to the second embodiment of the present invention.
FIG. 10 is a diagram illustrating image adjustment operation performed by a processing device according to a third embodiment of the present invention.
FIG. 11 is a diagram illustrating the image adjustment operation performed by the processing device according to the third embodiment of the present invention.
FIG. 12 is a diagram illustrating the image adjustment operation performed by the processing device according to the third embodiment of the present invention.
FIG. 13 is a flowchart illustrating image adjustment operation performed by a processing device according to a fourth embodiment of the present invention.
FIG. 14 is a diagram illustrating the image adjustment operation performed by the processing device according to the fourth embodiment of the present invention.
FIG. 15 is a diagram illustrating image adjustment operation performed by a processing device according to a modification of the fourth embodiment of the present invention.
FIG. 16 is a schematic diagram illustrating a configuration of a conventional ultrasound diagnosis system.

### Description of Embodiments

Modes for carrying out the present invention (hereinafter referred to as "embodiment(s)") will be described below with reference to the accompanying drawings. In the following description, reference will be made to an ultrasound endoscopic system including a medical observation apparatus for generating an ultrasound image based on ultrasound echo, but the present invention is not limited to these embodiments. The same reference signs are used to designate the same elements throughout the drawings.

### (First Embodiment)

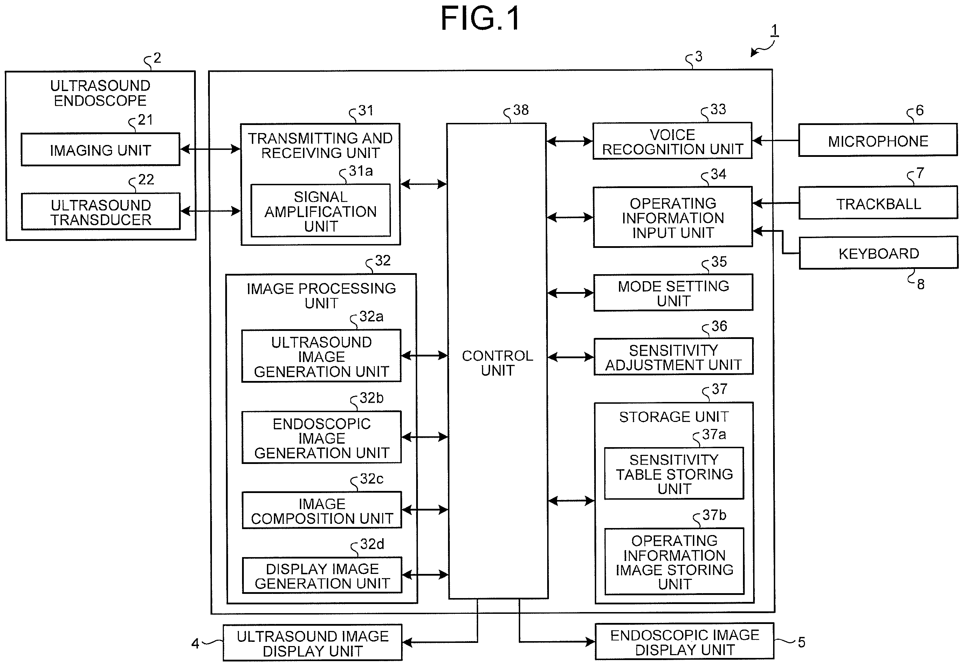

FIG. 1 is a block diagram illustrating a configuration of an ultrasound endoscopic system according to a first embodiment of the present invention. An ultrasound endoscopic system 1 illustrated in FIG. 1 is an apparatus for observing an observation target using an ultrasound wave.

The ultrasound endoscopic system 1 includes an ultrasound endoscope 2 for outputting an ultrasound pulse to receive reflected ultrasound echo, and imaging an imaging area including an area for outputting the ultrasound pulse to obtain an imaging signal, a processing device 3 for generating images respectively based on the ultrasound echo and the imaging signal obtained by the ultrasound endoscope 2, an ultrasound image display unit 4 for displaying various information including the image generated by the processing device 3 based on the ultrasound echo, an endoscopic image display unit 5 for displaying various information including the image generated by the processing device 3 based on the imaging signal, and a microphone 6, a trackball 7, and a keyboard 8 used for input of various instructions. The ultrasound image display unit 4 and the endoscopic image display unit 5 are achieved by the use of a liquid crystal display panel, an organic electro luminescence (EL) display panel, or the like.

The ultrasound endoscope 2 includes an insertion section having, at a distal end, an imaging unit 21 inserted into a body cavity of a subject to capture an in-vivo image of the subject, and an ultrasound transducer 22 for outputting the ultrasound pulse to the observation target, and receiving the ultrasound echo reflected from the observation target, and an operating unit for operating the imaging unit 21 and the ultrasound transducer 22.

The imaging unit 21 is achieved by the use of an image sensor having a two-dimensional arrangement of pixels, each of which is configured to receive light and to perform photoelectric conversion on the light to generate a signal. The image sensor includes a charge coupled device (CCD) image sensor or a complementary metal oxide semiconductor (CMOS) image sensor, for example.

Here, when the observation target is living tissue, the ultrasound transducer 22 may be used in any form of an external probe emitting an ultrasound wave from a surface of a living body, a miniature ultrasound probe including an elongated insertion section configured to be inserted into lumen such as digestive tract, biliopancreatic duct, or blood vessel, and an ultrasound endoscope further including an optical system in an intraluminal ultrasound probe. If the ultrasound endoscope is employed, the ultrasound transducer 22 is provided on a distal end side of the insertion section of the intraluminal ultrasound probe, and the intraluminal ultrasound probe is removably connected to the processing device on a proximal end side.

The ultrasound transducer 22 converts an electrical pulse signal received from a transmitting and receiving unit 31 to an ultrasound pulse (acoustic pulse signal), and converts the ultrasound echo reflected from an external specimen to an electrical echo signal. The ultrasound transducer 22 may have an ultrasound transducer performing mechanical scanning, or a plurality of ultrasound transducers performing electronic scanning.

The processing device 3 has the transmitting and receiving unit 31, an image processing unit 32, a voice recognition unit 33, an operating information input unit 34, a mode setting unit 35, a sensitivity adjustment unit 36, a storage unit 37, and a control unit 38.

The transmitting and receiving unit 31 transmits and receives electrical signals to and from the imaging unit 21 and the ultrasound transducer 22. The transmitting and receiving unit 31 is electrically connected with the imaging unit 21, transmits imaging information such as imaging timing to the imaging unit 21, and receives the imaging signal generated by the imaging unit 21. Furthermore, the transmitting and receiving unit 31 is electrically connected with the ultrasound transducer 22, transmits the electrical pulse signal to the ultrasound transducer 22, and receives the echo signal as an electrical reception signal from the ultrasound transducer 22. Specifically, the transmitting and receiving unit 31 generates the electrical pulse signal based on a preset waveform and transmission timing, and transmits the generated pulse signal to the ultrasound transducer 22.

The transmitting and receiving unit 31 has a signal amplification unit 31a for amplifying the echo signal. The signal amplification unit 31a performs sensitivity time control (STC) correction for amplifying an echo signal having a larger reception depth with a higher amplification factor. The transmitting and receiving unit 31 subjects the echo signal amplified by the signal amplification unit 31a to processing such as filtering, and then subjecting the echo signal to A/D conversion to generate and output a digital radio frequency (RF) signal in a time domain.

The image processing unit 32 generates endoscopic image data based on the imaging signal, and image data corresponding to the electrical echo signal. The image processing unit 32 has an ultrasound image generation unit 32a, an endoscopic image generation unit 32b, an image composition unit 32c, and a display image generation unit 32d.

The ultrasound image generation unit 32a generates B-mode image data as an ultrasound image displayed by converting an amplitude of the echo signal to a luminance. The ultrasound image generation unit 32a generates the B-mode image data by subjecting a digital signal to signal processing using a known technique such as a bandpass filter, logarithmic conversion, gain control, contrast processing, and to decimation or the like of data according to a data step width determined according to a display range of an image in the ultrasound image display unit 4. A B-mode image is a grayscale image having equal R (red), G (green), and B (blue) values, as variables, in an RGB color system employed as a color space. In the first embodiment, the B-mode image is an image of a segmented area partially segmented from a scan area from which ultrasound echo is received.

The endoscopic image generation unit 32b generates in-vivo image data displayed on the endoscopic image display unit 5, based on the imaging signal. The endoscopic image generation unit 32b subjects the imaging signal to predetermined image processing, and generates the in-vivo image data including the in-vivo image. The in-vivo image is a color image having R, G, and B values, as variables, in the RGB color system employed as the color space.

The image composition unit 32c for example generates composite image data including a composite image (display image) obtained by combining B-mode image data and an image (operating information image). The B-mode image data is generated by the ultrasound image generation unit 32a, and the image (operating information image) is displayed according to a signal input by the microphone 6, the trackball 7, or the like.

The display image generation unit 32d subjects the image data generated by the ultrasound image generation unit 32a, the endoscopic image generation unit 32b, or the image composition unit 32c to predetermined processing such as gradation processing, and then outputs a signal obtained after the processing as display image data to be displayed.

The voice recognition unit 33 detects a frequency of voice input to the microphone 6, compares the detected frequency of voice with previously stored feature data to obtain a language group, and receives the obtained language group as a recognition result (first operation instruction signal). Specifically, when an operator inputs voice "rotate" to the microphone 6, the voice recognition unit 33 recognizes "rotate", and outputs a command relating to "rotate" to the control unit 38, as a recognition result.

The operating information input unit 34 receives input of an operation instruction signal from the trackball 7 or the keyboard 8, and outputs the operation instruction signal to the control unit 38. For example, when the trackball 7 is operated, the operating information input unit 34 outputs, to the control unit 38, an instruction signal (second operation instruction signal) including a movement amount (rotation direction, rotation amount, or the like) of the trackball 7.

The mode setting unit 35 sets an image adjustment mode, based on the recognition result (command) of the voice recognition unit 33. Specifically, when the command relating to "rotate" is input from the voice recognition unit 33, the mode setting unit 35 sets the image adjustment mode to a "rotation mode". The mode setting unit 35 outputs information about the set image adjustment mode to the control unit 38.

The sensitivity adjustment unit 36 calculates image adjustment amounts according to the rotation direction or the rotation amount of the trackball 7 input to the operating information input unit 34, according to the image adjustment mode set by the mode setting unit 35. Specifically, when the image adjustment mode set by the mode setting unit 35 is the "rotation mode", the sensitivity adjustment unit 36 determines a rotation direction of the image according to the rotation direction of the trackball 7, and calculates a rotation amount of the image, based on the rotation amount of the trackball 7. The sensitivity adjustment unit 36 outputs the determined rotation direction and the calculated rotation amount to the control unit 38.

The storage unit 37 stores data or the like including various programs for operating the ultrasound endoscopic system 1, and various parameters required for operation of the ultrasound endoscopic system 1. The storage unit 37 has a sensitivity table storing unit 37a and an operating information image storing unit 37b.

The sensitivity table storing unit 37a is created according to a command (image adjustment mode) input from the voice recognition unit 33 to store image adjustment amounts, for example, the rotation direction and the rotation amount of the image, for each mode.

The operating information image storing unit 37b stores an operating information image (guide image) according to the image adjustment mode. The operating information image is an image representing a relationship between a direction of an operation in a contact signal input unit (trackball 7), and a direction of an operation according to a signal input function in a processing mode (image adjustment mode). For example, in the rotation mode in which the trackball 7 rotates an image, the operating information image represents an image for guiding a rotation direction relative to arrangement of an operator's hand and the trackball 7. The operating information image is combined with the B-mode image by the image composition unit 32c, and displayed on the ultrasound image display unit 4.

Furthermore, the storage unit 37 stores various programs including an operation program for executing an operation method of the ultrasound endoscopic system 1. The operation program can be recorded in a computer-readable recording medium, such as a hard disk, a flash memory, a CD-ROM, a DVD-ROM, or a flexible disk to be widely distributed. The above-mentioned various programs may be obtained by download via a communication network. Here, the communication network is achieved for example by an existing public switched telephone network, local area network (LAN), wide area network (WAN), or the like, which may be wired or wireless.

The storage unit 37 having the above-mentioned configuration is achieved by the use of a read only memory (ROM), a random access memory (RAM), and the like. The ROM previously installs the various programs or the like, and the RAM stores a calculation parameter, data, or the like for each processing.

The control unit 38 is achieved by the use of a central processing unit (CPU), various calculation circuits, or the like having a control function. The control unit 38 reads information stored in the storage unit 37 from the storage unit 37, and executes various calculation processing relating to the operation method of the ultrasound endoscopic system 1 to collectively controls the ultrasound endoscopic system 1.

The microphone 6 functions as a contactless operation unit according to the present invention, and converts voice (sound) of the operator to an electrical signal. Note that the word contactless represents non-presence of physical contact.

The trackball 7 is a pointing device functioning as a contact operation unit according to the present invention, and having a spherical body (ball). When the spherical body is rotated by the operation of the operator, a signal relating to a rotation direction, a rotation amount (or rotation speed), or the like of the spherical boy is output.

The keyboard 8 is provided with a plurality of keys, and a signal is output according to a pressed key. The keyboard 8 may be achieved by a plurality of keys on a touch panel, or may have a display unit for displaying an image.

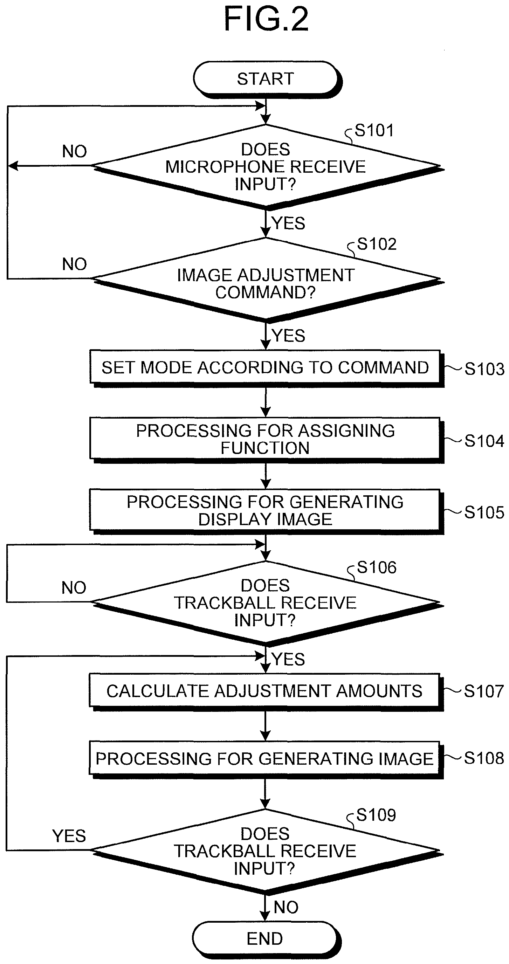

Next, image adjustment operation performed by the processing device 3 of the ultrasound endoscopic system 1 having the above-described configuration will be described with reference to the drawings. FIG. 2 is a flowchart illustrating the image adjustment operation performed by the processing device according to the first embodiment of the present invention.

First, the control unit 38 determines whether the microphone 6 receives voice input (step S101: contactless signal input step). When the control unit 38 obtains the recognition result from the voice recognition unit 33 (step S101: Yes), the process proceeds to step S102. In contrast, when the control unit 38 cannot obtain the recognition result from the voice recognition unit 33 (step S101: No), the process returns to step S101, and confirmation of the voice input from the microphone 6 is repeated.

When the process proceeds to step S102, the control unit 38 determines whether the recognition result from the voice recognition unit 33 relates to image adjustment. Here, the control unit 38 refers to a command output as the recognition result, and determines whether the command is a command relating to the image adjustment. When the control unit 38 determines that the command output as the recognition result is the command relating to the image adjustment (step S102: Yes), the process proceeds to step S103. In contrast, when the control unit 38 determines that the command output as the recognition result is not the command relating to the image adjustment (step S102: No), the process returns to step S101, and confirmation of the voice input from the microphone 6 is repeated.

In steps S101 and S102, when the recognition result cannot be obtained from the voice recognition unit 33 (step S101: No), or when the command output as the recognition result is not determined as the command relating to the image adjustment (step S102: No), processing for the image adjustment operation may be finished instead of returning to step S101.

In step S103, the control unit 38 controls the mode setting unit 35 to set the image adjustment mode according to the command output as the recognition result (mode setting step). For example, when the command relating to "rotate" is input from the voice recognition unit 33, the mode setting unit 35 sets the image adjustment mode to the "rotation mode". The mode setting unit 35 outputs information about the set image adjustment mode to the control unit 38.

When the information about the image adjustment mode is output from the mode setting unit 35, the control unit 38 subjects the signal input from the trackball 7 to processing for assigning a signal input function according to the set mode (step S104: function assignment step).

Then, the control unit 38 performs processing for generating the display image having the B-mode image and the operating information image (step S105: image processing step). In the processing for generating the display image, processing for image composition is performed so that the image composition unit 32c obtains an operating information image according to the set mode, with reference to the operating information image storing unit 37b, and the operating information image is displayed on the ultrasound image display unit 4 together with the B-mode image data.

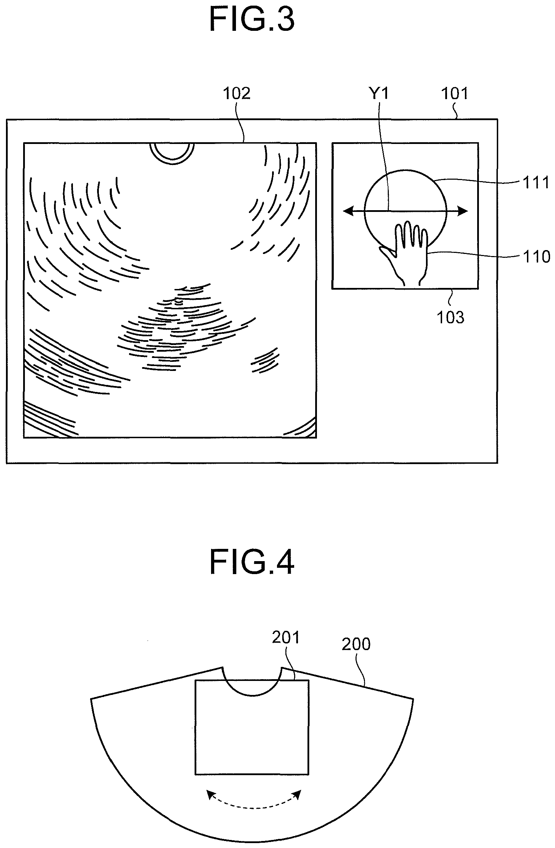

FIG. 3 is a diagram illustrating the image adjustment operation performed by the processing device according to the first embodiment of the present invention. In the image adjustment mode, a display area 101 displayed on the ultrasound image display unit 4 is provided with an ultrasound image display area 102 for displaying the ultrasound image (B-mode image), and an operating information image display area 103 for displaying the operating information image for guiding an image adjustment corresponding to the image adjustment mode (see FIG. 3). The operating information image indicating an operator's hand 110 and an image of the trackball 7 (trackball image 111) is displayed, in the operating information image display area 103. When the image adjustment mode is the "rotation mode", an arrow Y1 indicating an operation direction is displayed, in addition to the operator's hand 110 and the trackball image 111. The arrow Y1 indicates for example a reference direction set to determine a rotation direction and a rotation amount. Owing to the operating information image, a relationship between the position (direction) of the operator's hand 110 and the position of the trackball 7 (trackball image 111), and a moving direction of the operator's hand 110 can be visually confirmed.

After the processing for generating the display image, the control unit 38 determines whether the trackball 7 receives operation (input) (step S106). When the control unit 38 determines that the trackball 7 receives the operation (input) (step S106: Yes), the process proceeds to step S107. In contrast, when the control unit 38 determines that the trackball 7 does not receive the operation (input) (step S106: No), confirmation of the operation (input) of the trackball 7 is repeated.

When the control unit 38 determines that the trackball 7 receives the operation (input) (step S106: Yes), processing for calculating the image adjustment amounts is performed (step S107). In the processing for calculating the adjustment amounts, the sensitivity adjustment unit 36 calculates the image adjustment amounts according to the rotation direction or the rotation amount of the trackball 7, and the assigned signal input function. Specifically, when the image adjustment mode is set as the "rotation mode", the sensitivity adjustment unit 36 determines a rotation direction according to the rotation direction of the trackball 7, and calculates the rotation amount of the image based on the rotation amount of the trackball 7.

FIG. 4 is a diagram illustrating the image adjustment operation performed by the processing device according to the first embodiment of the present invention. The sensitivity adjustment unit 36 detects a direction and an amount of a component of the direction indicated by the arrow Y1, for the rotation direction of the trackball 7 rotated by the operator, and determines the rotation direction and calculates the rotation amount of the image, based on the detected direction and amount. Here, the ultrasound image displayed on the ultrasound image display area 102 is for example an area (e.g., segmented area 201 such as region of interest (ROI) or display area including the ROI) included in a scan area 200 from which ultrasound echo is received, as illustrated in FIG. 4. The rotation direction of the image determined by the sensitivity adjustment unit 36 is output for example as a clockwise direction or counterclockwise direction in a direction along an arc connecting the same depths in a scanning direction (arc about ultrasound oscillation source). The image (segmented area 201) may be rotated with the center as a rotation axis, or the scan area 200 may be rotated.

When information about the rotation direction and the rotation amount is output from the sensitivity adjustment unit 36, the control unit 38 performs image generation processing for generating an adjusted image, in step S108. In the image generation processing, the ultrasound image generation unit 32a changes the segmented area 201 according to the information about the rotation direction and the rotation amount, and generates a B-mode image according to the changed segmented area.

Then, the control unit 38 determines whether the trackball 7 receives operation (input) (step S109). When the control unit 38 determines that the trackball 7 receives the operation (input) (step S109: Yes), the process proceeds to step S107, and the above-mentioned processing for calculating the adjustment amounts is performed. In contrast, when the control unit 38 determines that the trackball 7 does not receive the operation (input) (step S109: No), the process ends. The image adjustment operation may be determined to be finished when the trackball 7 is not operated for a predetermined time, or may be determined to be finished when input (e.g., input of "finish the rotation") is made to finish the rotation mode through the microphone 6.

As described above, the B-mode image generated by the processing device 3 through the image adjustment operation is displayed on the ultrasound image display area 102 of the display area 101 displayed on the ultrasound image display unit 4. In the adjustment operation, the operator sets a mode by voice, and manually controls the rotation of an image while looking at the operating information displayed on the ultrasound image display unit 4. Therefore, the operator can perform operation while watching the ultrasound image display unit 4, and displacement of an observation position is prevented during operation such as image adjustment.

For confirmation of a moving direction of the operator's hand, a trajectory of the rotation direction may be displayed when the trackball 7 is operated. In this configuration, the trajectory having a fixed length is displayed. That is, only the rotation direction may be displayed, or the trajectory may have a length changed corresponding to the rotation amount.

According to the first embodiment described above, the mode setting unit 35 changes a mode based on the recognition result of the voice recognition unit 33, the operating information image is displayed on the ultrasound image display unit 4 according to a set mode to show the operation of the trackball 7, and the sensitivity adjustment unit 36 calculates the image adjustment amounts, according to the operation of the trackball 7. Thus, the adjustment operation of the image on the monitor can be performed while watching the monitor.

In the first embodiment, the hand 110 arranged on the lower side of the trackball image 111 is described, but the position of the hand 110 may be changed according to a layout or the like of the ultrasound endoscopic system 1. The arrangement of the hand 110 may have a plurality of patterns stored as the operating information image, and for example the arrangement of the hand is set before the image adjustment operation or upon activation of the system.

Further, the above-mentioned first embodiment has described that the reference direction is set to determine the adjustment amounts, and the adjustment amounts are calculated according to a component of the reference direction in the operation direction of the trackball 7, but the adjustment amounts may be determined according to an angle between the operation direction and the reference direction, and calculation of the adjustment amounts is not limited to the above description.

### (Modification of First Embodiment)

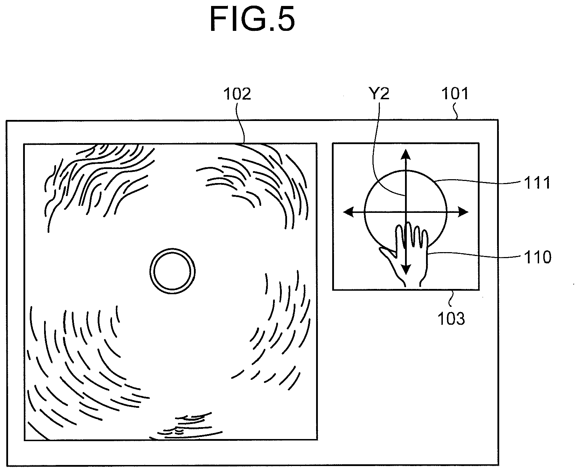

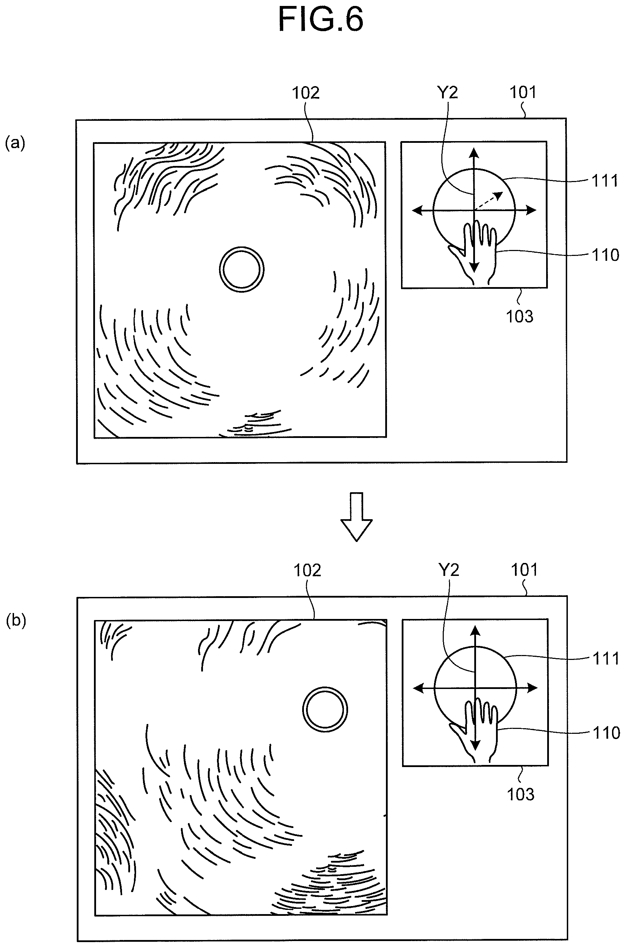

Next, a modification of the first embodiment of the present invention will be described with reference to the drawings. FIGS. 5 and 6 are diagrams illustrating image adjustment operation performed by a processing device according to the modification of the first embodiment of the present invention. FIG. 6(a) illustrates an image of the display area upon input of image adjustment, and FIG. 6(b) illustrates an image of the display area obtained after the image adjustment. In the above first embodiment, an example of voice instruction "rotate" has been described, but in the present modification, an example of voice instruction "scroll" will be described in which an ultrasound image (B-mode image) obtained by a radial ultrasound transducer is displayed. In the present modification, the above-described scan area 200 has an annular shape in which the ultrasound transducer is disposed at the center, and the segmented area 201 is a rectangular area positioned on the scan area.

In step S103 of the above-mentioned flowchart (see FIG. 2), when a command relating to "scroll" is input from the voice recognition unit 33, the mode setting unit 35 sets an image adjustment mode to a "scroll mode". The mode setting unit 35 outputs information about the set image adjustment mode to the control unit 38.

When the information about the image adjustment mode is output from the mode setting unit 35, the control unit 38 performs processing for assigning a signal input function according to the set mode (step S104), and processing for generating a display image having the B-mode image and an operating information image (step S105: image processing step). When the image adjustment mode is the "scroll mode (display area setting mode)", the operator's hand 110, the trackball image 111, and operation axes Y2 indicating an operation direction are displayed, as illustrated in FIG. 5. For example, the operation axes Y2 include two orthogonal axes indicating reference directions set to determine a moving direction and a movement amount of an image center position, respectively. The operation axes Y2 are axes set so that a moving direction of the segmented area 201 relative to the scan area 200 and a moving direction of an image in the ultrasound image display area 102 are coincident with each other.

Then, When the control unit 38 determines that the trackball 7 receives the operation (input) (step S106: Yes), the processing for calculating image adjustment amounts is performed (step S107). The sensitivity adjustment unit 36 detects directions and amounts of components of the rotation direction of the trackball 7 for the operation axes Y2, relative to a rotation direction of the trackball 7 rotated by the operator, and determines a scroll direction and calculates a scroll amount of the image, based on the detected directions and amounts. Here, the scroll direction of the image (segmented area 201) determined by the sensitivity adjustment unit 36 is output for example, as a moving direction (e.g., dotted arrow of FIG. 6(a)) from the image center position.

When information about the scroll direction and the scroll amount is output from the sensitivity adjustment unit 36, the control unit 38 performs the image generation processing for generating an adjusted image, in step S108. In the image generation processing, the ultrasound image generation unit 32a changes the segmented area 201 according to the information about the scroll direction and the scroll amount, and generates a B-mode image according to the changed segmented area (see FIG. 6(b)).

### (Second Embodiment)

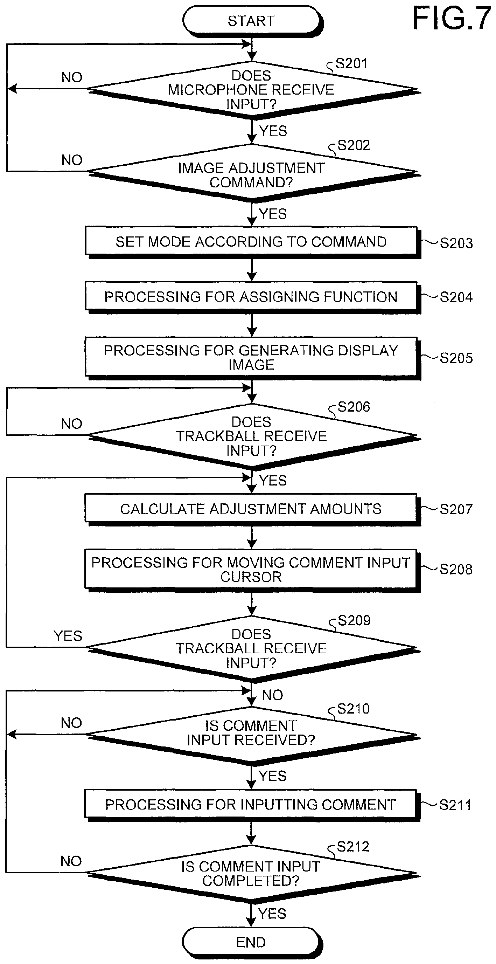

A second embodiment of the present invention will be described below. FIG. 7 is a flowchart illustrating image adjustment operation performed by a processing device according to the second embodiment of the present invention. The same reference signs are used to designate the same elements as those described above. In the above first embodiment, an example of voice instruction "rotate" has been described, but in the second embodiment, an example of voice instruction "comment" will be described.

First, the control unit 38 determines whether the microphone 6 receives voice input (step S201). When the control unit 38 obtains a recognition result from the voice recognition unit 33 (step S201: Yes), the process proceeds to step S202. In contrast, when the control unit 38 cannot obtain the recognition result from the voice recognition unit 33 (step S201: No), the process returns to step S201, and confirmation of the voice input from the microphone 6 is repeated.

When the process proceeds to step S202, the control unit 38 determines whether the recognition result from the voice recognition unit 33 relates to image adjustment. Here, the control unit 38 refers to a command output as the recognition result, and determines whether the command is a command relating to the image adjustment. When the control unit 38 determines that the command output as the recognition result is the command relating to the image adjustment (step S202: Yes), the process proceeds to step S203. In contrast, when the control unit 38 determines that the command output as the recognition result is not the command relating to the image adjustment (step S202: No), the process returns to step S201, and confirmation of the voice input from the microphone 6 is repeated.

In step S203, when a command relating to "comment" is input from the voice recognition unit 33, the mode setting unit 35 sets an image adjustment mode to "comment mode (text input mode)". The mode setting unit 35 outputs information about the set image adjustment mode to the control unit 38.

When the information about the image adjustment mode is output from the mode setting unit 35, the control unit 38 subjects a signal input from the trackball 7 to the processing for assigning a signal input function according to the set mode (step S204).

Then, the control unit 38 performs processing for generating a display image having the B-mode image and an operating information image (step S205). In the processing for generating the display image, processing for image composition is performed so that the image composition unit 32c obtains an operating information image according to the set mode, with reference to the operating information image storing unit 37b, and the operating information image is displayed on the ultrasound image display unit 4 together with the B-mode image data.

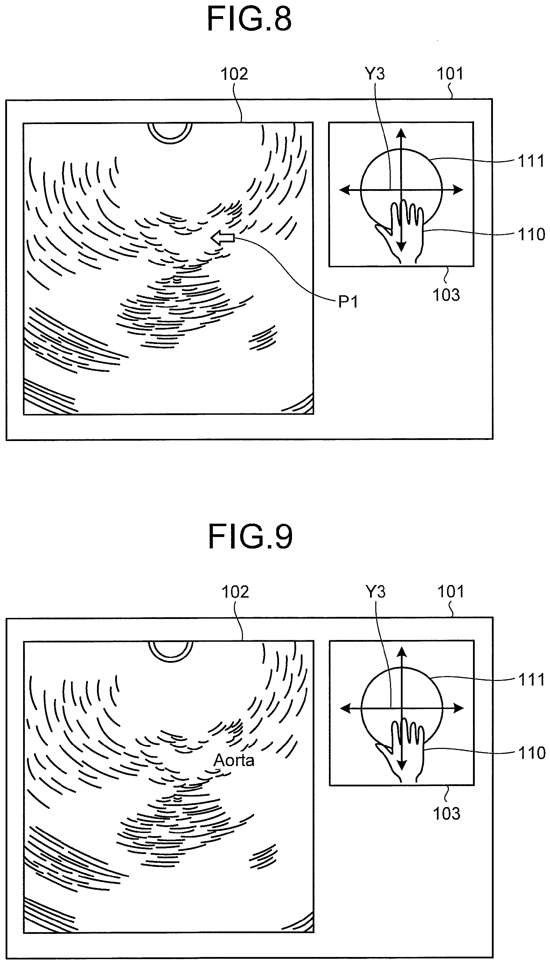

FIG. 8 is a diagram illustrating the image adjustment operation performed by the processing device according to the second embodiment. When the image adjustment mode is a "comment mode", the operator's hand 110, the trackball image 111, and operation axes Y3 indicating an operation direction are displayed, as illustrated in FIG. 8. For example, the operation axes Y3 include two orthogonal axes indicating reference directions set to determine a moving direction and a movement amount of a comment input cursor P1, respectively.

After the processing for generating the display image, the control unit 38 determines whether the trackball 7 receives operation (input) (step S206). When the control unit 38 determines that the trackball 7 receives the operation (input) (step S206: Yes), the process proceeds to step S207. In contrast, when the control unit 38 determines that the trackball 7 does not receive the operation (input) (step S206: No), confirmation of the operation (input) of the trackball 7 is repeated.

When the control unit 38 determines that the trackball 7 receives the operation (input) (step S206: Yes), processing for calculating adjustment amounts of the comment input cursor P1 is performed (step S207). The sensitivity adjustment unit 36 detects components of axis directions of the operation axes Y3 for a rotation direction and a rotation amount of the trackball 7 rotated by the operator, and calculates movement amounts in the axis directions based on the detected components. The sensitivity adjustment unit 36 outputs the calculated movement amounts in the axis directions as a movement amount of the comment input cursor P1. For the movement amount of the comment input cursor P1, the movement amounts in the axis directions may be output, or coordinates after movement in the B-mode image may be output as the adjustment amounts.

When information about the movement amount of the comment input cursor P1 is output from the sensitivity adjustment unit 36, the control unit 38 performs a processing for moving the comment input cursor P1 in the B-mode image, in step S208. In the processing for moving, the ultrasound image generation unit 32a moves the comment input cursor P1 in the B-mode image, according to the information about the movement amount of the comment input cursor P1, and generates a B-mode image generated after the movement. The control unit 38 displays the B-mode image generated after the movement, on the ultrasound image display unit 4 (ultrasound image display area 102).

Then, the control unit 38 determines whether the trackball 7 receives operation (input) (step S209). When the control unit 38 determines that the trackball 7 receives the operation (input) (step S209: Yes), the process proceeds to step S207, and the above-mentioned processing for moving the comment input cursor P1 is performed. In contrast, when the control unit 38 determines that the trackball 7 does not receive the operation (input) (step S209: No), the process proceeds to step S210.

In step S210, it is determined whether voice is input (comment input) from the microphone 6. After the image adjustment mode is set to the "comment mode", textual information (character string) according to voice input from the microphone 6 is processed as an input comment. When the control unit 38 obtains the recognition result from the voice recognition unit 33 (step S210: Yes), the process proceeds to step S211. In contrast, when the control unit 38 cannot obtain the recognition result from the voice recognition unit 33 (step S210: No), confirmation of the voice input from the microphone 6 is repeated.

In step S211, processing for inputting the comment is performed according to the recognition result from the voice recognition unit 33. FIG. 9 is a diagram illustrating the image adjustment operation performed by the processing device according to the second embodiment of the present invention. In the processing for inputting the comment, the control unit 38 compares the recognition result from the voice recognition unit 33 and languages previously stored to output a comparison result, and the image composition unit 32c performs processing for combining the character string based on the comparison result. Specifically, when a voice "Aorta" is input, a character string "Aorta" is inserted according to a position of the comment input cursor P1 (see FIG. 8), as illustrated in FIG. 9. Voice recognition may be finished upon recognition of a voice "set" or "convert", and a list of conversion candidates may be displayed upon recognition of the voice "convert". Selection of a conversion candidate may be determined by voice input of a number applied to each conversion candidate, or may be performed by operation of the trackball 7.

Then, the control unit 38 determines whether the processing for inputting the comment is completed (step S212). When the control unit 38 receives a signal representing completion of comment input, through predetermined comment input completion operation (step S212: Yes), the process ends. In contrast, when the control unit 38 does not receive the signal representing completion of the comment input, for example, additional input operation of a comment (step S212: No), the process returns to step S210, and the processing for inputting the comment is repeated.

According to the second embodiment, the mode setting unit 35 changes a mode based on the recognition result of the voice recognition unit 33, the operating information image is displayed on the ultrasound image display unit 4 according to the set mode to show the operation of the trackball 7, and the sensitivity adjustment unit 36 calculates the movement amount of the comment input cursor P1 according to the operation of the trackball 7, and the processing for inputting the comment is performed by voice recognition after movement of the comment input cursor P1. Thus, the adjustment operation of the image on the monitor can be performed while watching the monitor.

### (Third Embodiment)

Next, a third embodiment of the present invention will be described. The same reference signs are used to designate the same elements as those described above, in the drawings according to the third embodiment. In the above first embodiment, an example of voice instruction "rotate" has been described, but in the third embodiment, an example of voice instruction "focus position" will be described. Image adjustment operation of the third embodiment is performed according to the flowchart illustrated in FIG. 2.

In the third embodiment, a command relating to "focus position" is input from the voice recognition unit 33, in step S103. When the command relating to "focus position" is input from the voice recognition unit 33, the mode setting unit 35 sets an image adjustment mode to a "focus position change mode". The mode setting unit 35 outputs information about the set image adjustment mode to the control unit 38.

When the information about the image adjustment mode is output from the mode setting unit 35, the control unit 38 performs processing for assigning a signal input function according to the set mode (step S104), and processing for generating a display image having the B-mode image and an operating information image (step S105: image processing step).

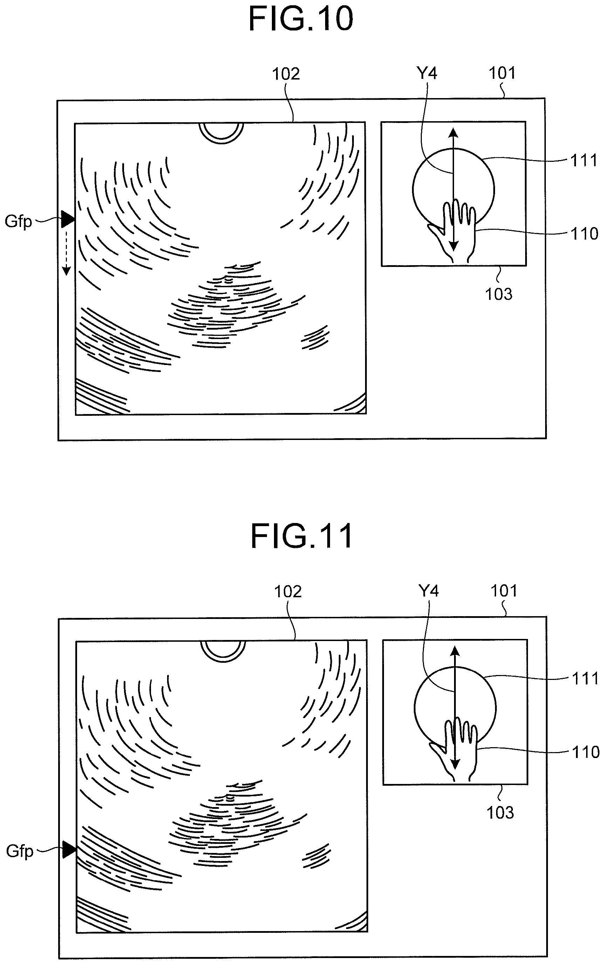

FIGS. 10 and 11 are diagrams illustrating the image adjustment operation performed by a processing device according to the third embodiment. When the image adjustment mode is the "focus position change mode", an arrow Y4 indicating an operation direction is displayed in addition to the operator's hand 110 and the trackball image 111. The arrow Y4 indicates for example a reference direction set to determine a focus position. Furthermore, the display area 101 displays focus position information Gfp for indicating a focus position of an image displayed on the ultrasound image display area 102. The focus position information Gfp indicates for example a position corresponding to a depth, along a vertical direction of the ultrasound image display area 102.

When the control unit 38 determines that the trackball 7 receives the operation (input) (step S106: Yes), processing for changing the focus position is performed (step S107). In the processing for changing the focus position, the sensitivity adjustment unit 36 calculates an amount of change in focus position, according to a rotation direction or a rotation amount of the trackball 7. Specifically, when the image adjustment mode is set as the "focus position change mode", the sensitivity adjustment unit 36 determines the rotation direction according to the rotation direction of the trackball 7, and calculates the amount of change in focus position, based on the rotation amount of the trackball 7.

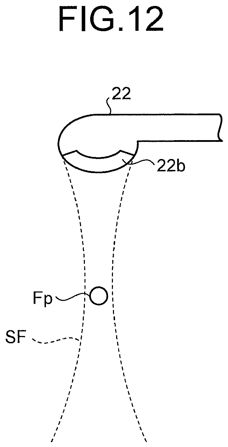

FIG. 12 is a diagram illustrating the image adjustment operation performed by the processing device according to the third embodiment. As illustrated in FIG. 12, a sound wave generator 22b provided in the ultrasound transducer 22 forms a sound field SF having a shape substantially symmetrical about the focus position Fp in a traveling direction of an ultrasound wave (vertical direction in FIG. 12). The sensitivity adjustment unit 36 detects a direction and amount of a component of the direction indicated by the arrow Y4, relative to the rotation direction of the trackball 7 rotated by the operator, and determines a moving direction of the focus position Fp and calculates a movement amount thereof, based on the detected direction and amount.

When information about the moving direction and the movement amount of the focus position Fp is output from the sensitivity adjustment unit 36, the control unit 38 performs the image generation processing for generating an adjusted image, in step S108. In the image generation processing according to the third embodiment, irradiation of an ultrasound wave is performed at a changed focus position Fp to receive ultrasound echo, and the ultrasound image generation unit 32a generates a new B-mode image according to the changed focus position Fp (see FIG. 11). Furthermore, the display area 101 illustrated in FIG. 11 displays the focus point Gfp positioned according to the changed focus position, which is changed from the focus position illustrated in FIG. 10.

Then, the control unit 38 determines whether the trackball 7 receives operation (input) (step S109). When the control unit 38 determines that the trackball 7 receives the operation (input) (step S109: Yes), the process proceeds to step S107, and the above-mentioned processing for changing the focus position Fp is performed. In contrast, when the control unit 38 determines that the trackball 7 does not receive the operation (input) (step S109: No), the process ends.

According to the third embodiment, the mode setting unit 35 changes a mode based on a recognition result of the voice recognition unit 33, the operating information image is displayed on the ultrasound image display unit 4 according to a set mode to show the operation of the trackball 7, and the sensitivity adjustment unit 36 calculates an amount of change in focus position Fp according to the operation of the trackball 7. Thus, the adjustment operation of the image on the monitor can be performed while watching the monitor.

### (Fourth Embodiment)

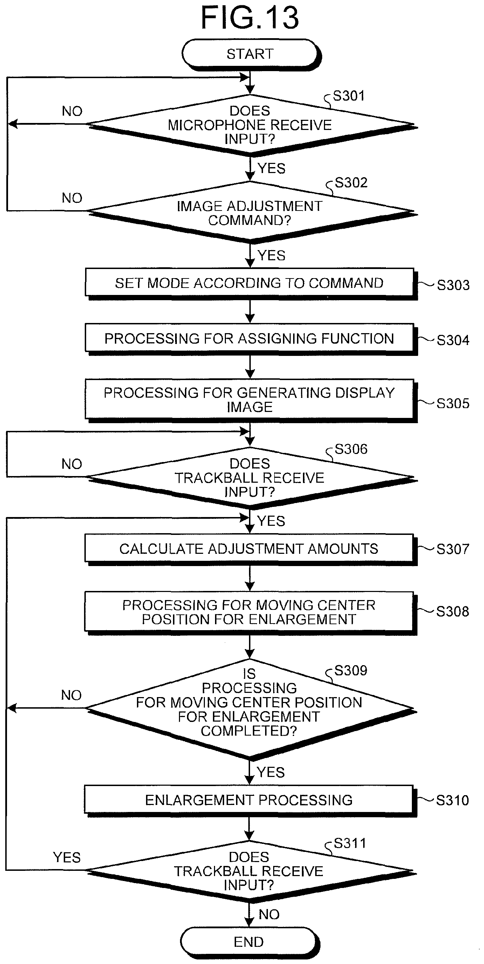

Next, a fourth embodiment of the present invention will be described. FIG. 13 is a flowchart illustrating image adjustment operation performed by a processing device according to the fourth embodiment of the present invention. The same reference signs are used to designate the same elements as those described above. In the above first embodiment, an example of voice instruction "rotate" has been described, but in the fourth embodiment, an example of voice instruction "enlarge" will be described.

First, the control unit 38 determines whether the microphone 6 receives voice input (step S301). When the control unit 38 obtains a recognition result from the voice recognition unit 33 (step S301: Yes), the process proceeds to step S302. In contrast, when the control unit 38 cannot obtain the recognition result from the voice recognition unit 33 (step S301: No), the process returns to step S301, and confirmation of the voice input from the microphone 6 is repeated.

When the process proceeds to step S302, the control unit 38 determines whether the recognition result from the voice recognition unit 33 relates to image adjustment. Here, the control unit 38 refers to a command output as the recognition result, and determines whether the command is a command relating to the image adjustment. When the control unit 38 determines that the command output as the recognition result is the command relating to the image adjustment (step S302: Yes), the process proceeds to step S303. In contrast, when the control unit 38 determines that the command output as the recognition result is not the command relating to the image adjustment (step S302: No), the process returns to step S301, and confirmation of the voice input from the microphone 6 is repeated.

In step S303, when a command relating to "enlarge" is input from the voice recognition unit 33, the mode setting unit 35 sets an image adjustment mode to an "enlargement mode". The mode setting unit 35 outputs information about the set image adjustment mode to the control unit 38.

When the information about the image adjustment mode is output from the mode setting unit 35, the control unit 38 subjects a signal input from the trackball 7 to processing for assigning a signal input function according to the set mode (step S304).

Then, the control unit 38 performs processing for generating a display image having a B-mode image and an operating information image (step S305). In the processing for generating the display image, processing for image composition is performed so that the image composition unit 32c obtains an operating information image according to the set mode, with reference to the operating information image storing unit 37b, and the operating information image is displayed on the ultrasound image display unit 4 together with B-mode image data.

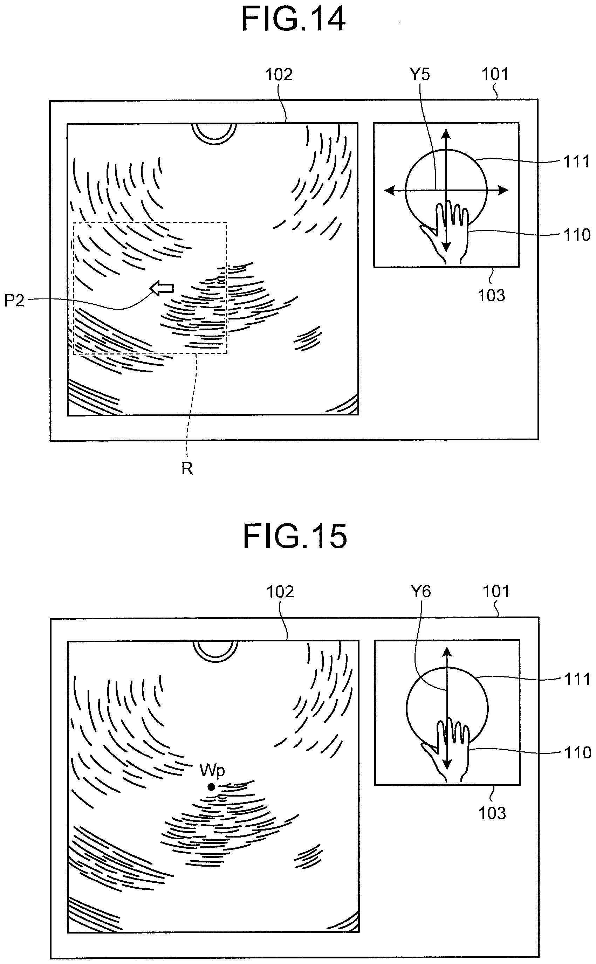

FIG. 14 is a diagram illustrating the image adjustment operation performed by the processing device according to the fourth embodiment of the present invention. When the image adjustment mode is the "enlargement mode", the operator's hand 110, the trackball image 111, and operation axes Y5 indicating an operation direction are displayed, as illustrated in FIG. 14. For example, the operation axes Y5 include two orthogonal axes indicating reference directions set to determine a moving direction and a movement amount of an enlargement position instruction cursor P2 for indicating a center position for enlargement, respectively.

When the mode setting unit 35 sets the image adjustment mode, the control unit 38 determines whether the trackball 7 receives operation (input) (step S306). When the control unit 38 determines that the trackball 7 receives the operation (input) (step S306: Yes), the process proceeds to step S307. In contrast, when the control unit 38 determines that the trackball 7 does not receive the operation (input) (step S306: No), confirmation of the operation (input) of the trackball 7 is repeated.

When the control unit 38 determines that the trackball 7 receives the operation (input) (step S306: Yes), processing for calculating the movement amounts of the enlargement position instruction cursor P2 as adjustment amounts is performed (step S307). The sensitivity adjustment unit 36 detects components of axis directions of the operation axes Y5 for a rotation direction and a rotation amount of the trackball 7 rotated by the operator, and calculates movement amounts in the axis directions based on the detected components. The sensitivity adjustment unit 36 outputs the calculated movement amounts in the axis directions as the movement amount of the enlargement position instruction cursor P2. For the movement amount of the enlargement position instruction cursor P2, the movement amounts in the axis directions may be output, or coordinates after movement in the B-mode image may be output as the adjustment amounts.

When information about the movement amount of the enlargement position instruction cursor P2 is output from the sensitivity adjustment unit 36, the control unit 38 performs a processing for moving the enlargement position instruction cursor P2 in the B-mode image, in particular, a processing for moving the center position for enlargement, in step S308. In the processing for moving, the ultrasound image generation unit 32a moves the enlargement position instruction cursor P2 in the B-mode image, according to the information about the movement amount of the enlargement position instruction cursor P2, and generates a B-mode image generated after the movement. The control unit 38 displays the B-mode image generated after the movement, on the ultrasound image display unit 4 (ultrasound image display area 102).

Then, the control unit 38 determines whether the processing for moving the enlargement position instruction cursor P2 is completed (step S309). When the control unit 38 receives a signal representing completion of the processing for moving the enlargement position instruction cursor P2, through predetermined movement completion operation (step S309: Yes) the process proceeds to step S310. In contrast, when the control unit 38 does not receive the signal representing completion of the processing for moving the enlargement position instruction cursor P2, for example, additional operation (input) of the trackball 7 (step S309: No), the process returns to step S307, and the processing for calculating the movement amounts of the enlargement position instruction cursor P2 is repeated.

In step S310, enlargement processing is performed about a position instructed by the enlargement position instruction cursor P2. The ultrasound image generation unit 32a sets an area R for enlargement (see FIG. 14) according to a predetermined enlargement ratio, about the position instructed by the enlargement position instruction cursor P2, and trims the set area R for enlargement. The ultrasound image generation unit 32a generates a B-mode image in which the area R for enlargement is enlarged into a size according to the ultrasound image display area 102.

Then, the control unit 38 determines whether the trackball 7 receives operation (input) (step S311). When the control unit 38 determines that the trackball 7 receives the operation (input) (step S311: Yes), the process proceeds to step S307, and the above-mentioned processing for moving the enlargement position instruction cursor P2 in the B-mode image is performed. In contrast, when the control unit 38 determines that the trackball 7 does not receive the operation (input) (step S311: No), the process ends.

According to the fourth embodiment, the mode setting unit 35 changes a mode based on the recognition result of the voice recognition unit 33, the operating information image is displayed on the ultrasound image display unit 4 according to the set mode to show the operation of the trackball 7, and the sensitivity adjustment unit 36 calculates the movement amount of the enlargement position instruction cursor P2 according to the operation of the trackball 7, and the enlargement processing for enlarging the B-mode image is performed after movement of the enlargement position instruction cursor P2. Thus, the adjustment operation of the image on the monitor can be performed while watching the monitor.

### (Modification of Fourth Embodiment)

Next, a modification of the fourth embodiment of the present invention will be described with reference to the drawings. FIG. 15 is a diagram illustrating image adjustment operation performed by a processing device according to the modification of the fourth embodiment of the present invention. The above-mentioned fourth embodiment has described that the trackball 7 determines the center of enlargement to enlarge the B-mode image at the predetermined enlargement ratio, but in the present modification, a center of enlargement Wp is previously set, and an enlargement ratio is changed by operating the trackball 7.

In the present modification, when an enlargement mode is set as an image adjustment mode, the sensitivity adjustment unit 36 calculates, based on an operation direction Y6, the enlargement ratio according to the rotation direction and the rotation amount of the trackball 7. Furthermore, the control unit 38 determines whether a B-mode image currently displayed in the ultrasound image display area 102 is a frozen image or a live image of the B-mode image. When the control unit 38 determines that the B-mode image currently displayed in the ultrasound image display area 102 is the frozen image, processing for trimming the area for enlargement is performed about the set center of enlargement to have a size according to the calculated enlargement ratio, and an enlarged B-mode image is generated.

In contrast, when the control unit 38 determines that the B-mode image currently displayed in the ultrasound image display area 102 is the live B-mode image, a range is changed according to the enlargement ratio, and then ultrasound echo is additionally received with the previously set center of enlargement as a focus position. The ultrasound image generation unit 32a generates a B-mode image having a range according to the predetermined enlargement ratio, based on the received ultrasound echo.

In addition, image adjustment may be performed by combining the present modification and the above-mentioned fourth embodiment. Specifically, when the B-mode image currently displayed in the ultrasound image display area 102 is the frozen image, the processing of steps S304 to S306 is performed to determine a center position for trimming, and trimming is performed about the center position different from the center for enlargement previously set.

Furthermore, in the above-described first to fourth embodiments, the microphone 6 (voice input) for voice detection has been described as the contactless operation unit, but the contactless operation unit may detect gesture to output a recognition result, eye gaze to output a recognition result, or a brain wave to output a recognition result. When the contactless operation unit (e.g., IR) for recognition of the gesture is used, a gesture recognition unit is provided instead of the voice recognition unit 33 to output a command as the recognition result, according to gesture. If a contactless operation unit for eye-gaze recognition (e.g., a unit for imaging eyes (eye gaze)) is employed, an eye-gaze recognition unit is provided instead of the voice recognition unit 33 to output a command as the recognition result, according to the eye gaze (gaze movement).

In the above-mentioned first to fourth embodiments, the rotation mode, the scroll mode, the focus position change mode, and the enlargement mode have been exemplified, but, in addition to them, processing modes for observation, such as a B-mode image reduction mode, a region-of-interest setting mode for setting a region of interest, a measurement mode for designating a measuring side line or a measuring area to measure a predetermined region in the B-mode image, may be set through the microphone 6 and operated by the trackball 7.

In addition, in the above-mentioned first to fourth embodiments, the trackball 7 has been described as the contact operation unit, but a keyboard, a mouse, a touch panel, a lever, a dial, a joystick, a foot switch, or the like may be used as the contact operation unit, or two or more of them may be combined. For example, when the contact operation unit employs the touch panel, an operating information image such as a hand, a touch panel, or an operation direction may be displayed on the operating information image display area 103 to perform operation, such as tapping, dragging, pinching in, or pinching out, for intuitive instruction of image adjustment. The contact operation unit is preferably an input unit for instructing continuous or discontinuous, or multistep or stepless switching adjustment as a signal input function in the image adjustment mode. Here, the continuous switching adjustment represents time-series continuous signal input, and the instruction of discontinuous switching adjustment instruction represents time-series discontinuous signal input (including intermittent signal input for image confirmation or the like by operator). In addition, the instruction of stepless switching adjustment represents the signal output according to continuous input operation such as rotation of the trackball 7, and the instruction of multistep switching adjustment represents the signal output according to intermittent input operation such as pressing of a button of the keyboard or click of the mouse.

Furthermore, in the above-mentioned first to fourth embodiments, the ultrasound image display unit 4 and the endoscopic image display unit 5 have been described to display respective images, but one display unit may be employed to have a display area divided into areas in which an ultrasound image (including the operating information image) and an endoscopic image are displayed.

Furthermore, in the ultrasound endoscopic system 1 according to the present invention, the image adjustment modes according to the above-mentioned first to fourth embodiments are appropriately switched according to the input from the microphone 6, and further the operating information image is switched according to the switched image adjustment mode to observe an observation target. The position of the trackball 7 may be changed to switch the operating information image (position of the hand 110 relative to the trackball image 111).

Furthermore, the above-mentioned first to fourth embodiments has exemplified the ultrasound endoscope observing the living tissue as the observation target, but the above-mentioned first to fourth embodiments may be applied to an industrial endoscope for observing characteristics of a material. Furthermore, an observation apparatus including the ultrasound transducer 22, the processing device 3 (excluding endoscopic image generation unit 32b), the ultrasound image display unit 4, the microphone 6, and the trackball 7 may be employed, in addition to the endoscope. The observation apparatus according to the present invention can be applied to both inside and outside a body. Instead of the ultrasound wave, the observation target may be irradiated with infrared light to transmit and receive a signal of the observation target.

As described above, the present invention may include various embodiments without departing from the technical scope of the invention as set forth in the appended claims.

### Industrial Applicability

As described above, a medical observation apparatus, a method for operating the medical observation apparatus, and a program for operating the medical observation apparatus according to the present invention are useful for an adjustment operation of an image on a monitor, while watching the monitor.

### Reference Signs List

- 1: ULTRASOUND ENDOSCOPIC SYSTEM
- 2: ULTRASOUND ENDOSCOPE
- 3: PROCESSING DEVICE
- 4: ULTRASOUND IMAGE DISPLAY UNIT
- 5: ENDOSCOPIC IMAGE DISPLAY UNIT
- 6: MICROPHONE
- 7: TRACKBALL
- 8: KEYBOARD
- 21: IMAGING UNIT
- 22: ULTRASOUND TRANSDUCER
- 31: TRANSMITTING AND RECEIVING UNIT
- 32: IMAGE PROCESSING UNIT
- 32a: ULTRASOUND IMAGE GENERATION UNIT
- 32b: ENDOSCOPIC IMAGE GENERATION UNIT
- 32c: IMAGE COMPOSITION UNIT
- 32d: DISPLAY IMAGE GENERATION UNIT
- 33: VOICE RECOGNITION UNIT
- 34: OPERATING INFORMATION INPUT UNIT
- 35: MODE SETTING UNIT
- 36: SENSITIVITY ADJUSTMENT UNIT
- 37: STORAGE UNIT
- 37a: SENSITIVITY TABLE STORING UNIT
- 37b: OPERATING INFORMATION IMAGE STORING UNIT
- 38: CONTROL UNIT

## Claims

1. A medical observation apparatus (1) that is configured to acquire a signal for generating an image of an observation target and to display an observation image based on the acquired signal, the medical observation apparatus (1) comprising:
a contactless signal input unit (6) configured to receive input of a first operation instruction signal through a contactless operation by an operator;
a contact signal input unit (7, 8) configured to receive input of a second operation instruction signal through a contact operation by the operator;
a control unit (38) configured to set at least one of processing modes for the observation image according to the first operation instruction signal, and to assign at least one of signal input functions according to the at least one of the processing modes to the second operation instruction signal that is input to the contact signal input unit (7, 8); and
an image processing unit (32) configured to generate a display image having the observation image and having at least one of guide images for guiding an operation according to the at least one of the signal input functions assigned to the contact signal input unit (7, 8),
**characterized in that**
each of the guide images being an image representing a relationship between a position of a hand of the operator who operates the contact signal input unit (7, 8), and a direction of movement of the observation image according to the at least one of the signal input functions in the at least one of the processing modes set by the control unit (38).

2. The medical observation apparatus (1) according to claim 1, wherein
the control unit (38) is configured to control switching between the processing modes according to input of the first operation instruction signal, switching between the guide images according to the processing modes, or switching between the signal input functions according to the processing modes.

3. The medical observation apparatus (1) according to claim 1, wherein
the contactless signal input unit (6) is configured to detect voice, gesture, eye gaze, or a brain wave, and
the contact signal input unit (7, 8) includes at least one of a keyboard, a trackball, a mouse, a touch panel, a lever, a dial, a joystick, and a foot switch.

4. The medical observation apparatus (1) according to claim 1, wherein
the signal input functions according to the processing modes include continuous or discontinuous, or multistep or stepless contact operation.

5. The medical observation apparatus (1) according to claim 1, wherein
the processing modes include at least one of a rotation mode for rotating an image, a display area setting mode for setting a display area for image display within an observation area, a text input mode, a focus position setting mode, an enlargement or reduction mode, a region-of-interest setting mode, and a measurement mode.

6. The medical observation apparatus (1) according to claim 1, wherein
the signal for generating the image of the observation target is a signal generated based on an echo signal that is obtained by converting an ultrasound echo into an electrical signal, the ultrasound echo being obtained by transmitting an ultrasound wave to the observation target and by reflecting the ultrasound wave from the observation target.

7. A method for operating a medical observation apparatus (1) that is configured to acquire a signal for generating an image of an observation target and to display an observation image based on the acquired signal, the method comprising:
a contactless signal input step of receiving, by a contactless signal input unit (6), input of a first operation instruction signal through a contactless operation by an operator;
a mode setting step of setting, by a control unit (38), at least one of processing modes for the observation image according to the first operation instruction signal;
a function assignment step of assigning, by the control unit (38), at least one of signal input functions according to the at least one of the processing modes set in the mode setting step, to a contact signal input unit (7, 8) for receiving input of a second operation instruction signal through a contact operation by the operator; and
an image processing step of generating, by an image processing unit (32), a display image having the observation image and having at least one of guide images,
**characterized in that**
each of the guide images being an image representing a relationship between a position of a hand of the operator who operates the contact signal input unit (7, 8), and a direction of movement of the observation image according to the at least one of the signal input functions in the at least one of the processing modes set by the control unit (38).

8. A program for operating a medical observation apparatus (1) that is configured to acquire a signal for generating an image of an observation target and to display an observation image based on the acquired signal, the program causing the medical observation apparatus (1) to execute:
a contactless signal input step of receiving, by a contactless signal input unit (6), input of a first operation instruction signal through a contactless operation by an operator;
a mode setting step of setting, by a control unit (38), at least one of processing modes for the observation image according to the first operation instruction signal;
a function assignment step of assigning, by the control unit (38), at least one of signal input functions according to the at least one of the processing modes set in the mode setting step, to a contact signal input unit (7, 8) for receiving input of a second operation instruction signal through a contact operation by the operator; and
an image processing step of generating, by an image processing unit (32), a display image having the observation image and having at least one of guide images,
**characterized in that**
each of the guide images being an image representing a relationship between a position of a hand of the operator who operates the contact signal input unit (7, 8), and a direction of movement of the observation image according to the at least one of the signal input functions in the at least one of the processing modes set by the control unit (38).

## Patentansprüche

1. Medizinische Beobachtungsvorrichtung (1), die so eingerichtet ist, dass sie ein Signal zum Erzeugen eines Bildes eines Beobachtungsziels erfasst und ein Beobachtungsbild auf der Grundlage des erfassten Signals anzeigt, wobei die medizinische Beobachtungsvorrichtung (1) umfasst:
eine berührungslose Signaleingabeeinheit (6), die so eingerichtet ist, dass sie die Eingabe eines ersten Betriebsanweisungssignals durch eine berührungslose Betätigung durch einen Bediener empfängt;
eine Kontaktsignaleingabeeinheit (7, 8), die so eingerichtet ist, dass sie die Eingabe eines zweiten Betriebsanweisungssignals durch eine Kontaktbetätigung durch den Bediener empfängt;
eine Steuereinheit (38), die so eingerichtet ist, dass sie mindestens einen von Verarbeitungsmodi für das Beobachtungsbild gemäß dem ersten Betriebsanweisungssignal einstellt und mindestens eine von Signaleingabefunktionen gemäß dem mindestens einen der Verarbeitungsmodi dem zweiten Betriebsanweisungssignal zuordnet, das in die Kontaktsignaleingabeeinheit (7, 8) eingegeben wird; und
eine Bildverarbeitungseinheit (32), die so eingerichtet ist, dass sie ein Anzeigebild mit dem Beobachtungsbild und mit mindestens einem von Führungsbildern zum Führen einer Betätigung gemäß der mindestens einen der Signaleingabefunktionen, die der Kontaktsignaleingabeeinheit (7, 8) zugewiesen sind, erzeugt,
**dadurch gekennzeichnet, dass**
jedes der Führungsbilder ein Bild ist, das eine Beziehung zwischen einer Position einer Hand des Bedieners, der die Kontaktsignaleingabeeinheit (7, 8) betätigt, und einer Bewegungsrichtung des Beobachtungsbildes gemäß der mindestens einen der Signaleingabefunktionen in dem mindestens einen der von der Steuereinheit (38) eingestellten Verarbeitungsmodi darstellt.

2. Medizinische Beobachtungsvorrichtung (1) nach Anspruch 1, wobei
die Steuereinheit (38) so eingerichtet ist, dass sie Umschalten zwischen den Verarbeitungsmodi in Abhängigkeit von der Eingabe des ersten Betriebsanweisungssignals, Umschalten zwischen den Führungsbildern in Abhängigkeit von den Verarbeitungsmodi oder Umschalten zwischen den Signaleingabefunktionen in Abhängigkeit von den Verarbeitungsmodi steuert.

3. Medizinische Beobachtungsvorrichtung (1) nach Anspruch 1, wobei
die berührungslose Signaleingabeeinheit (6) so eingerichtet ist, dass sie Sprache, Gesten, Blicke oder Gehirnströme erkennt, und
die Kontaktsignaleingabeeinheit (7, 8) mindestens eines der folgenden Elemente umfasst: eine Tastatur, einen Trackball, eine Maus, ein Berührungsfeld, einen Hebel, einen Drehknopf, einen Joystick und einen Fußschalter.

4. Medizinische Beobachtungsvorrichtung (1) nach Anspruch 1, wobei
die Signaleingangsfunktionen entsprechend den Verarbeitungsmodi kontinuierliche oder diskontinuierliche, oder mehrstufige oder stufenlose Kontaktbetätigung umfasst.

5. Medizinische Beobachtungsvorrichtung (1) nach Anspruch 1, wobei
die Verarbeitungsmodi mindestens einen von einem Rotationsmodus zum Drehen eines Bildes, einen Anzeigebereichseinstellungsmodus zum Einstellen eines Anzeigebereichs für die Bildanzeige innerhalb eines Beobachtungsbereichs, einen Texteingabemodus, einen Fokuspositionseinstellungsmodus, einen Vergrößerungs- oder Verkleinerungsmodus, einen Modus zum Einstellen des interessierenden Bereichs und einen Messmodus umfasst.

6. Medizinische Beobachtungsvorrichtung (1) nach Anspruch 1, wobei
das Signal zum Erzeugen des Bildes des Beobachtungsziels ein Signal ist, das auf der Grundlage eines Echosignals erzeugt wird, das durch Umwandeln eines Ultraschallechos in ein elektrisches Signal erhalten wird, wobei das Ultraschallecho durch Senden einer Ultraschallwelle zu dem Beobachtungsziel und durch Reflektieren der Ultraschallwelle von dem Beobachtungsziel erhalten wird.

7. Verfahren zum Betreiben einer medizinischen Beobachtungsvorrichtung (1), das so eingerichtet ist, dass es ein Signal zur Erzeugung eines Bildes eines Beobachtungsziels erfasst und ein Beobachtungsbild auf der Grundlage des erfassten Signals anzeigt, wobei das Verfahren umfasst:
einen Schritt einer berührungslosen Signaleingabe, bei dem durch eine berührungslose Signaleingabeeinheit (6) ein erstes Betriebsanweisungssignal durch eine berührungslose Bedienung von einem Bediener eingegeben wird;
einen Moduseinstellungsschritt zum Einstellen mindestens eines von Verarbeitungsmodi für das Beobachtungsbild gemäß dem ersten Betriebsanweisungssignal durch eine Steuereinheit (38);
einen Funktionszuweisungsschritt eines Zuweisens, durch die Steuereinheit (38), mindestens einer von Signaleingabefunktionen gemäß dem mindestens einen der Verarbeitungsmodi, die in dem Moduseinstellungsschritt eingestellt sind, an eine Kontaktsignaleingabeeinheit (7, 8) zum Empfangen der Eingabe eines zweiten Betriebsanweisungssignals durch eine Kontaktbetätigung durch den Bediener; und
einen Bildverarbeitungsschritt eines Erzeugens, durch eine Bildverarbeitungseinheit (32), eines Anzeigebildes mit dem Beobachtungsbild und mit mindestens einem der Führungsbilder,
**dadurch gekennzeichnet, dass**
jedes der Führungsbilder ein Bild ist, das eine Beziehung zwischen einer Position einer Hand des Bedieners, der die Kontaktsignaleingabeeinheit (7, 8) betätigt, und einer Bewegungsrichtung des Beobachtungsbildes gemäß der mindestens einen der Signaleingabefunktionen in dem mindestens einen der von der Steuereinheit (38) eingestellten Verarbeitungsmodi darstellt.

8. Programm zum Betreiben einer medizinische Beobachtungsvorrichtung (1), das so eingerichtet ist, dass es ein Signal zur Erzeugung eines Bildes eines Beobachtungsziels erfasst und ein Beobachtungsbild auf der Grundlage des erfassten Signals anzeigt, wobei das Programm bewirkt, dass die medizinische Beobachtungsvorrichtung ausführt (1):
einen Schritt einer berührungslosen Signaleingabe, bei dem durch eine berührungslose Signaleingabeeinheit (6) ein erstes Betriebsanweisungssignal durch eine berührungslose Bedienung von einem Bediener eingegeben wird;
einen Moduseinstellungsschritt zum Einstellen mindestens eines von Verarbeitungsmodi für das Beobachtungsbild gemäß dem ersten Betriebsanweisungssignal durch eine Steuereinheit (38);
einen Funktionszuweisungsschritt eines Zuweisens, durch die Steuereinheit (38), mindestens einer von Signaleingabefunktionen gemäß dem mindestens einen der Verarbeitungsmodi, die in dem Moduseinstellungsschritt eingestellt sind, an eine Kontaktsignaleingabeeinheit (7, 8) zum Empfangen einer Eingabe eines zweiten Betriebsanweisungssignals durch eine Kontaktbetätigung durch den Bediener; und
einen Bildverarbeitungsschritt eines Erzeugens, durch eine Bildverarbeitungseinheit (32), eines Anzeigebildes mit dem Beobachtungsbild und mit mindestens einem der Führungsbilder,
**dadurch gekennzeichnet, dass**
jedes der Führungsbilder ein Bild ist, das eine Beziehung zwischen einer Position einer Hand des Bedieners, der die Kontaktsignaleingabeeinheit (7, 8) betätigt, und einer Bewegungsrichtung des Beobachtungsbildes gemäß der mindestens einen der Signaleingabefunktionen in dem mindestens einen der von der Steuereinheit (38) eingestellten Verarbeitungsmodi darstellt.

## Revendications

1. Appareil d'observation médicale (1) qui est conçu pour acquérir un signal destiné à générer une image d'un cible d'observation et pour afficher une image d'observation se basant sur le signal acquis, l'appareil d'observation médicale (1) comprenant :
une unité d'entrée de signal sans contact (6) conçue pour recevoir l'entrée d'un premier signal d'instruction de fonctionnement par un fonctionnement sans contact par un utilisateur ;
une unité d'entrée de signal par contact (7, 8) conçue pour recevoir l'entrée d'un second signal d'instruction de fonctionnement par un fonctionnement par contact par un utilisateur ;
une unité de commande (38) conçue pour définir au moins l'un des modes de traitement pour l'image d'observation conformément au premier signal d'instruction de fonctionnement, et pour attribuer au moins l'une des fonctions d'entrée de signal conformément à au moins un des modes de traitement au second signal d'instruction de fonctionnement qui est entré dans l'unité d'entrée de signal par contact (7, 8) ;
une unité de traitement d'images (32) conçue pour générer une image d'affichage ayant l'image d'observation et ayant au moins l'une des images guides pour guider un fonctionnement conformément à ladite fonction parmi les fonctions d'entrée de signal attribuée à l'unité d'entrée de signal par contact (7, 8),
**caractérisé en ce que**
chacune des images guides étant une image représentant une relation entre une position d'une main de l'utilisateur qui fait fonctionner l'unité d'entrée de signal par contact (7, 8), et un sens de mouvement de l'image d'observation conformément à ladite fonction parmi les fonctions d'entrée de signal dans ledit mode parmi les modes de traitement défini par l'unité de commande (38).

2. Appareil d'observation médicale (1) selon la revendication 1, dans lequel
l'unité de commande (38) étant conçue pour commander la commutation entre les modes de traitement conformément à l'entrée du premier signal d'instruction de fonctionnement, pour commuter entre les images guides conformément aux modes de traitement, ou pour commuter entre les fonctions d'entrée de signal conformément aux modes de traitement.

3. Appareil d'observation médicale (1) selon la revendication 1, dans lequel
l'unité d'entrée de signal sans contact (6) étant conçue pour détecter la voix, un geste, un regard, ou un mouvement cérébral, et
l'unité d'entrée de signal par contact (7, 8) comprend au moins un élément parmi un clavier, une boule de commande, une souris, un écran tactile, un levier, un rotateur, un manche et une pédale.

4. Appareil d'observation médicale (1) selon la revendication 1, dans lequel
les fonctions d'entrée de signal conformément aux modes de traitement comprennent le fonctionnement par contact sans étape, à plusieurs étapes, continu ou discontinu.

5. Appareil d'observation médicale (1) selon la revendication 1, dans lequel
les modes de traitement comprennent un mode de rotation pour tourner une image et/ou un mode de définition de zone d'affichage pour définir une zone d'affichage pour l'affichage d'image dans une zone d'observation et/ou un mode d'entrée de texte et/ou un mode de définition de position du foyer et/ou un mode de réduction ou d'agrandissement et/ou un mode de définition de région d'intérêt et/ou un mode de mesure.

6. Appareil d'observation médicale (1) selon la revendication 1, dans lequel
le signal pour générer l'image de la cible d'observation est un signal généré sur la base d'un signal d'écho qui est obtenu par conversion d'un écho ultrasonore en un signal électrique, l'écho ultrasonore étant obtenu par transmission d'une onde ultrasonore à la cible d'observation et par réflexion de l'onde ultrasonore par la cible d'observation.

7. Procédé de fonctionnement d'un appareil d'observation médicale (1) qui est conçu pour acquérir un signal destiné à générer une image d'un cible d'observation et pour afficher une image d'observation se basant sur le signal acquis, le procédé comprenant :
une étape d'entrée de signal sans contact pour recevoir, par une unité d'entrée de signal sans contact (6), l'entrée d'un premier signal d'instruction de fonctionnement par un fonctionnement sans contact par un utilisateur ;
une étape de définition de mode pour définir, par une unité de commande (38) au moins l'un des modes de traitement pour l'image d'observation conformément au premier signal d'instruction de fonctionnement ;
une étape d'attribution de fonction pour attribuer, par l'unité de commande (38), au moins l'une des fonctions d'entrée de signal conformément audit mode parmi les modes de traitement dans l'étape de définition de mode, à une unité d'entrée de signal par contact (7, 8) pour recevoir l'entrée d'un second signal d'instruction de fonctionnement par un fonctionnement par contact de l'utilisateur ; et
une étape de traitement d'image pour générer, par une unité de traitement d'image (32), une image d'affichage ayant l'image d'observation et ayant au moins l'une des images guides,
**caractérisé en ce que**
chacune des images guides étant une image représentant une relation entre une position d'une main de l'utilisateur qui fait fonctionner l'unité d'entrée de signal par contact (7, 8), et un sens de mouvement de l'image d'observation conformément à ladite fonction parmi les fonctions d'entrée de signal dans ledit mode parmi les modes de traitement défini par l'unité de commande (38).

8. Programme de fonctionnement d'un appareil d'observation médicale (1) qui est conçu pour acquérir un signal destiné à générer une image d'un cible d'observation et pour afficher une image d'observation se basant sur le signal acquis, le programme amenant l'appareil d'observation médicale (1) à exécuter :
une étape d'entrée de signal sans contact de réception, par une unité d'entrée de signal sans contact (6), de l'entrée d'un premier signal d'instruction de fonctionnement par un fonctionnement sans contact d'un utilisateur ;
une étape de définition de mode pour définir, par une unité de commande (38) au moins l'un des modes de traitement pour l'image d'observation conformément au premier signal d'instruction de fonctionnement ;
une étape d'attribution de fonction pour attribuer, par l'unité de commande (38), au moins l'une des fonctions d'entrée de signal conformément audit mode parmi les modes de traitement dans l'étape de définition de mode, à une unité d'entrée de signal par contact (7, 8) pour recevoir l'entrée d'un second signal d'instruction de fonctionnement par un fonctionnement par contact de l'utilisateur ; et
une étape de traitement d'image pour générer, par une unité de traitement d'image (32), une image d'affichage ayant l'image d'observation et ayant au moins l'une des images guides,
**caractérisé en ce que**
chacune des images guides étant une image représentant une relation entre une position d'une main de l'utilisateur qui fait fonctionner l'unité d'entrée de signal par contact (7, 8), et un sens de mouvement de l'image d'observation conformément à ladite fonction parmi les fonctions d'entrée de signal dans ledit mode parmi les modes de traitement défini par l'unité de commande (38).
